# EUROPEAN PATENT SPECIFICATION

(11) **EP 1 193 252 B1**
(45) Date of publication and mention of the grant of the patent: **27.12.2006**
(21) Application number: 02000688.8
(22) Date of filing: 22.09.1992
(51) Int. Cl.: C07D 205/08, C07F 7/18

(54) **Beta-Lactams useful for preparation of substituted isoserine esters using metal alkoxides**
Beta-Lactame verwendbar zur Herstellung von substituierten Isoserinestern mit Metallalkoxiden
Béta-Lactames utilsables dans la prépartion d'ester substitués d'isosérine en utilisant de oxydes de métaux

(30) Priority: 23.09.1991 US 763805; 03.04.1992 US 862955; 06.04.1992 US 863849
(43) Date of publication of application: 03.04.2002
(62) Divisional of application: 98114788.7
(73) Proprietor: FLORIDA STATE UNIVERSITY, Tallahassee, FL 32306-2763 (US)
(72) Inventor: Holton, Robert A., Tallahasse, Florida 32312 (US)
(74) Representative: W.P. Thompson & Co.

(56) References cited:
- EP-A- 0 400 971
- C. PALOMO, ET AL.: "Highly stereoselective synthesis of alpha-hydroxy beta-amino acids through beta-lactams: application to the synthesis of the taxol and bestatin side chains and related systems" TETRAHEDRON LETTERS, vol. 31, no. 44, 22 October 1990 (1990-10-22), pages 6429-6432, XP002224788 Elsevier Science Publishers, Amsterdam, NL ISSN: 0040-4039
- I. OJIMA, ET AL.: "A highly efficient route to tacotere by the beta-lactam synthon method" TETRAHEDRON LETTERS, vol. 34, no. 76, 25 June 1993 (1993-06-25), pages 4149-4152, XP000561213 Elsevier Science Publishers, Amsterdam, NL ISSN: 0040-4039
- I. OJIMA, ET AL.: "Synthesis and structure-activity relationships of novel nor-seco analogues of taxol and taxotere" JOURNAL OF ORGANIC CHEMISTRY, vol. 59, no. 3, 11 February 1994 (1994-02-11), pages 515-517, XP001094357 American Chemical Society, Washington, DC, US ISSN: 0022-3263
- F. FRAPPIER, ET AL.: "Alcaloïdes peptidiques - X. Approche de la synthèse des alcaloïdes peptidiques 1. Réactivité de N-tosylaziridines vis-à-vis de réactifs nucleophiles" TETRAHEDRON, vol. 34, no. 19, 1978, pages 2911-2916, XP002224841 Elsevier Science Publishers, Amsterdam, NL ISSN: 0040-4020
- H. AOYAMA, ET AL.: "Photochemical reactions of N-benzoylformyl-N- (phenylthiocarbonyl)amines. A novel photocyclisation involving 1,4-phenylthio migration" TETRAHEDRON LETTERS, vol. 24, no. 11, 1983, pages 1169-1170, XP002224840 Elsevier Science Publishers, Amsterdam, NL ISSN: 0040-4039
- G. JUST, ET AL.: "beta-Lactams. XI. Synthesis of N-phosphorylated mono- and bicyclic beta-lactams" CANADIAN JOURNAL OF CHEMISTRY., vol. 61, no. 8, 1983, pages 1730-1735, XP002224842 National Research Council, Ottawa, CA ISSN: 0008-4042

## Description

### BACKGROUND OF THE INVENTION

Three esters of N-acyl phenyl isoserine, taxol, taxotere and cephalomannine have been found to possess significant properties as antitumor agents.

This application describes a process for the preparation of N-acyl, N-sulfonyl and N-phosphoryl substituted isoserine esters, in general and to a semi-synthesis for the preparation of taxane derivatives such as taxol, taxotere and other biologically active derivatives involving the use of metal alkoxides and n-lactams, in particular.

The taxane family of terpenes, of which taxol is a member, has attracted considerable interest in both the biological and chemical arts.

Taxol is a promising cancer chemotherapeutic agent with a broad spectrum of antileukemic and tumor-inhibiting activity. Taxol has the following structure: 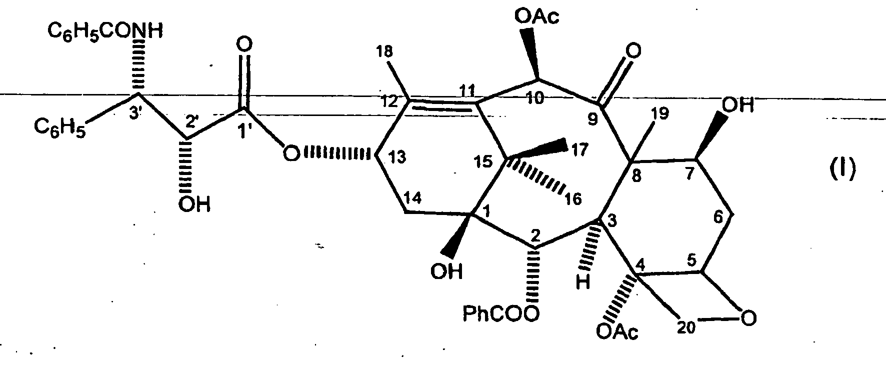 wherein Ph is phenyl and Ac is acetyl. Because of this promising activity, taxol is currently undergoing clinical trials in both France and the United States.

The supply of taxol for these clinical trials is presently being provided by the bark from Taxus brevifollia (Western Yew). However, taxol is found only in minute quantities in the bark of these slow growing evergreens, causing considerable concern that the limited supply of taxol will not meet the demand. Consequently, chemists in recent years have expended their energies in trying to find a viable synthetic route for the preparation of taxol. So far, the results have not been entirely satisfactory.

One synthetic route that has been proposed is directed to the synthesis of the tetracyclic taxane nucleus from commodity chemicals. A synthesis of the taxol congener taxusin has been reported by Holton, et al. in JACS 110, 6558 (1988). Despite the progress made in this approach, the final total synthesis of taxol is, nevertheless, likely to be a multi-step, tedious, and costly process.

A semi-synthetic approach to the preparation of taxol has been described by Greene, et al. in JACS 110, 5917 (1986), and involves the use of a congener of taxol, 10-deacetyl baccatin III which has the structure of formula II shown below: 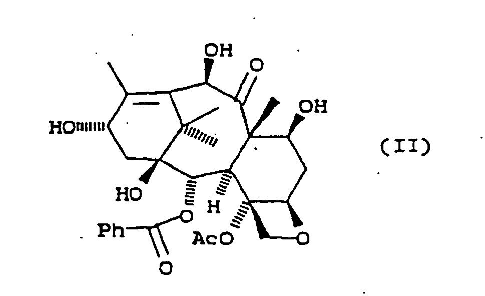 10-deacetyl baccatin III is more readily available than taxol since it can be obtained from the needles of Taxus baccata. According to the method of Greene et al., 10-deacetyl baccatin III is converted to taxol by attachment of the C-10 acetyl group and by attachment of the C-13 β-amido ester side chain through the esterification of the C-13 alcohol with a β-amido carboxylic acid unit. Although this approach requires relatively few steps, the synthesis of the β-amido carboxylic acid unit is a multi-step process which proceeds in low yield, and the coupling reaction is tedious and also proceeds in low yield. However, this coupling reaction is a key step which is required in every contemplated synthesis of taxol or biologically active derivative of taxol, since it has been shown by Wani, et al. in JACS 93, 2325 (1971) that the presence of the β-amido ester side chain at C13 is required for anti-tumor activity.

More recently, it has been reported in Colin et al. U.S. Patent No. 4,814,470 that taxol derivatives of the formula III below, have an activity significantly greater than that of taxol (I). 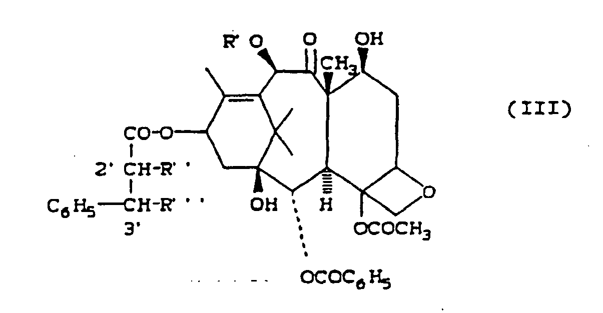 R' represents hydrogen or acetyl and one of R" and R"' represents hydroxy and the other represents tert-butoxycarbonylamino and their stereoisomeric forms, and mixtures thereof.

According to Colin et al., U.S. Patent 4,418,470, the products of general formula (III) are obtained by the action of the sodium salt of tert-butyl N-chlorocarbamate on a product of general formula: 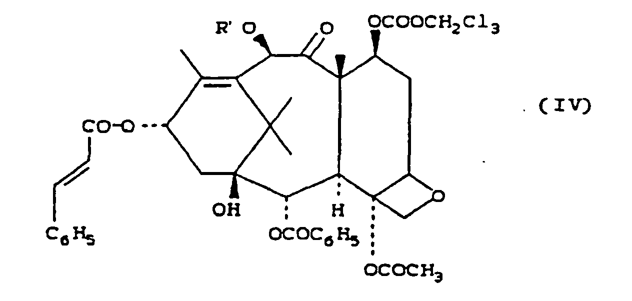 in which R' denotes an acetyl or 2,2,2-trichloroethoxycarbonyl radical, followed by the replacement of the 2,2,2-trichloroethoxycarbonyl group or groups by hydrogen. It is reported by Denis et al. in U.S. Patent No. 4,924,011, however, that this process leads to a mixture of isomers which has to be separated and, as a result, not all the baccatin III or 10-deactylbaccatin III employed for the preparation of the product of general formula (IV) can be converted to a product of general formula (III).

In an effort to improve upon the Colin et al. process, Denis et al. disclose a different process for preparing derivatives of baccatin III or of 10-deactylbaccatin III of general formula 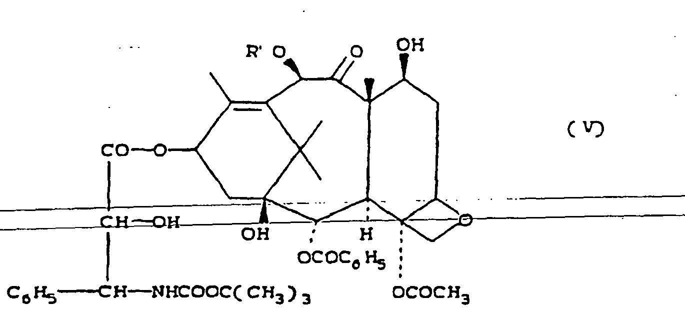 in which R' denotes hydrogen or acetyl wherein an acid of general formula: 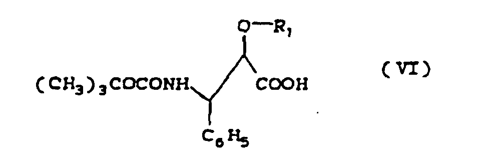 in which R₁ is a hydroxy-protecting group, is condensed with a taxane derivative of general formula: 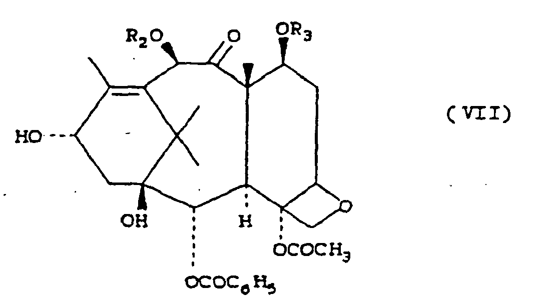 in which R₂ is an acetyl hydroxy-protecting group and R₃ is a hydroxy-prolecting group, and the protecting groups R₁, R₃ and, where appropriate, R₂ are then replaced by hydrogen. However, this method employs relatively harsh conditions, proceeds with poor conversion, and provides less than optimal yields.

A major difficulty remaining in the synthesis of taxol and other potential anti-tumor agents is the lack of a readily available method for easy attachment, to the C-13 oxygen, of the chemical unit which provides the β-amido ester side chain. Development of such a process for its attachment in high yield would facilitate the synthesis of taxol as well as related anti-tumor agents having a modified set of nuclear substituents or a modified C-13 side chain. This need has been fulfilled by the discovery of a new, efficient process for attachment, to the C-13 oxygen, of the chemical unit which provides the β-amido ester side chain.

Another major difficulty encountered in the synthesis of taxol is that known processes for the attachment of the β-amido ester side chain at C-13 are generally not sufficiently diastereoselective. Therefore the side chain precursor must be prepared in optically active form to obtain the desired diastereomer during attachment. The process of this invention, however, is highly diastereoselective, thus permitting the use of a racemic mixture of side chain precursor, eliminating the need for the expensive, time-consuming process of separating the precursor into its respective enantiomeric forms. The reaction additionally proceeds at a faster rate than previous processes, thus permitting the use of less side-chain precursor than has been required by such previous processes.

European Patent Publication No. 0400971 discloses β-lactams of the formula 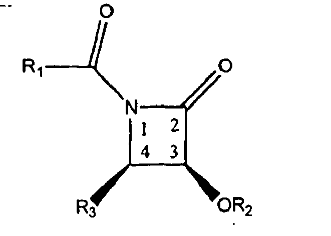 wherein R₁ is aryl, substituted aryl, alkyl, alkenyl, or alkynyl; R₂ is hydrogen, alkyl, acyl, acetal, ethoxyethyl, or other hydroxy protecting group; and R₄ is aryl, substituted aryl, alkyl, alkenyl or alkynyl.

### SUMMARY OF THE INVENTION

The present invention relates to a β-lactam having the formula: 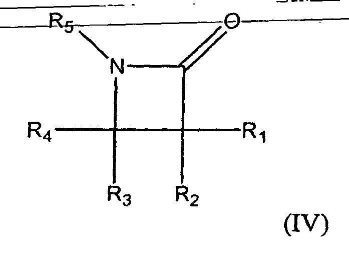 wherein
a β-lactam having the formula 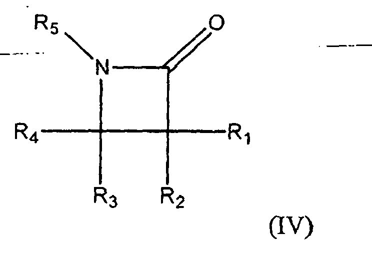 R₁ is -OR₆, -SR₇, or -NR₈R₉;
R₂ is hydrogen, alkyl, alkenyl, alkynyl, aryl, or heteroaryl;
R₃ and R₄ are independently hydrogen, alkyl, alkenyl, alkynyl, aryl, heteroaryl, or acyl, provided, however, that R₃ and R₄ are not both acyl;
R₅ is -COOR₁₀, -CONR₈R₁₀, or -SO₂R₁₁;
R₆ is hydrogen, alkyl, alkenyl, alkynyl, aryl, heteroaryl, hydroxy protecting group, or afunctional group which increases the water solubility of a taxane derivative; R₇ is alkyl, alkenyl, alkynyl, aryl, heteroaryl, or sulfhydryl protecting group;
R₈ is hydrogen, alkyl, alkenyl, alkynyl, aryl, or heteroaryl;
R₉ is an amino protecting group;
R₁₀ is alkyl;
R₁₁ is alkyl, alkenyl, alkynyl, aryl, heteroaryl, -OR₁₀, or -R₈R₁₄; and
R₁₄ is hydrogen, alkyl, alkenyl, alkynyl, aryl, or heteroaryl.

These β-lactams are useful in the production of isoserine esters. The isoserine esters are a side chain precursor for the synthesis of taxane derivatives which may be attached in relatively high yield to provide an intermediate which is readily converted to the desired taxane derivative.

Isoserine esters having the formula 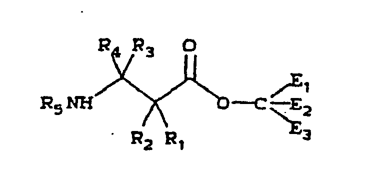 are obtained by reacting a β-lactam with a metal alkoxide, the β-lactam having the formula 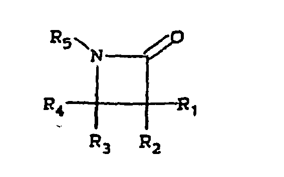 and the metal alkoxide having the formula
MOCE₁E₂E₃
wherein R₁ to R₅ are as previously defined.
E₁, E₂ and E₃ are independently hydrogen, hydrocarbon or cyclic, provided, at least one of E₁, E₂ and
E₃ is other than hydrogen. Preferably, two of E₁, E₂, and
E₃ together with the carbon to which they are attached comprise a mono- or polycyclic skeleton.

The metal alkoxide and β-lactam are selected so as to provide a process for preparing taxol, taxotere and other biologically active taxane derivatives having the following structural formula: 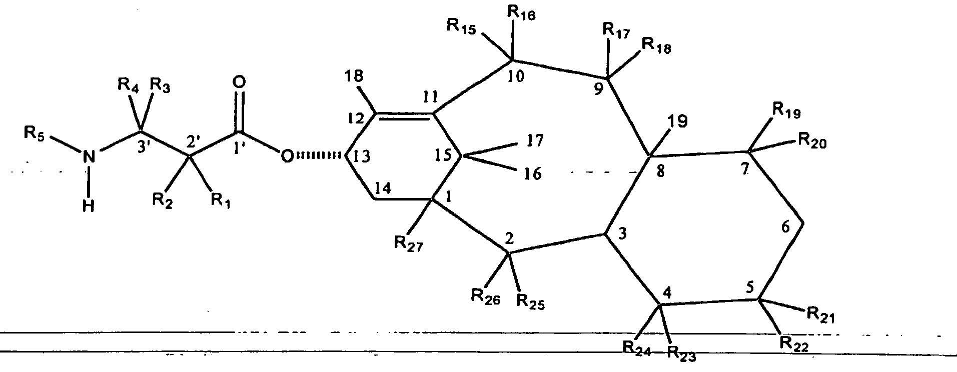 wherein
R₁ - R₁₄ are as previously defined,
R₁₅ and R₁₆ are independently hydrogen, hydroxy, lower alkanoyloxy, alkenoyloxy, alkynoyloxy, aryloyloxy or R₁₅ and R₁₆ together form an oxo;
R₁₇ and R₁₈ are independently hydrogen or lower alkanoyloxy, alkenoyloxy, alkynoyloxy, or aryloyloxy or R₁₇ and R₁₈ together form an oxo;
R₁₉ and R₂₀ are independently hydrogen or hydroxy or lower alkanoyloxy, alkenoyloxy, alkynoyloxy, or aryloyloxy;
R₂₁ and R₂₂ are independently hydrogen or lower alkanoyloxy, alkenoyloxy, alkynoyloxy, or aryloyloxy or R₂₁ and R₂₂ together form an oxo;
R₂₃ and R₂₄ are independently hydrogen or hydroxy or lower alkanoyloxy, alkenoyloxy, alkynoyloxy, or aryloyloxy; or
R₂₃ and R₂₄ together form an oxo or methylene or
R₂₃ and R₂₄ together with the carbon atom to which they are attached form an oxirane ring or
R₂₃ and R₂₂ together with the carbon atom to which they are attached form an oxetane ring;
R₂₅ is hydrogen, hydroxy, or lower alkanoyloxy, alkenoyloxy, alkynoyloxy, or aryloyloxy or
R₂₆ is hydrogen, hydroxy, or lower alkanoyloxy, alkenoyloxy, alkynoyloxy, or aryloyloxy; or R₂₆ and R₂₅ taken together form an oxo; and
R₂₇ is hydrogen, hydroxy or lower alkoxy, alkanoyloxy, alkenoyloxy, alkynoyloxy, or aryloyloxy.

Briefly, therefore, the taxane derivatives are prepared by reacting a β-lactam (2) with a metal alkoxide having the bi-, tri- or tetracyclic taxane nucleus to form a β-amido ester intermediate. The intermediate is then converted to the taxane derivative. β-lactam (2) has the general formula: 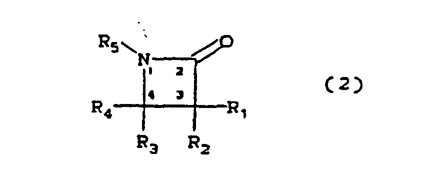 wherein R₁ - R₅ are as previously defined. The metal alkoxide preferably has the tricyclic taxane nucleus corresponding to the general formula: 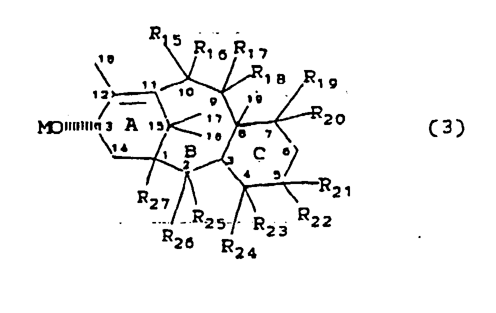 wherein M is a metal, and R₁₅-R₂₇ are as previously defined. Most preferably, the metal alkoxide has the tetracyclic taxane nucleus corresponding to metal alkoxide (3) wherein R₂₂ and R₂₃ together form an oxetane ring.

Other objects and features of this invention will be in part apparent and in part pointed out hereinafter.

### DETAILED DESCRIPTION

The present invention is directed to a process for preparing substituted isoserine esters, in general, and taxol, taxotere and other taxane derivatives which are biologically active using β-lactam (2), the structure of which is depicted hereinbelow: 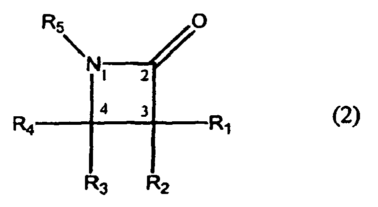 wherein R₁, R₂, R₃, R₄ and R₅ are as previously defined.

In accordance with the present invention,
R₂ and R₄ are hydrogen or lower alkyl. R₃ is preferably aryl, most preferably, naphthyl, phenyl, 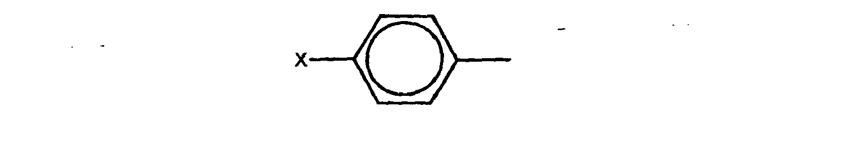 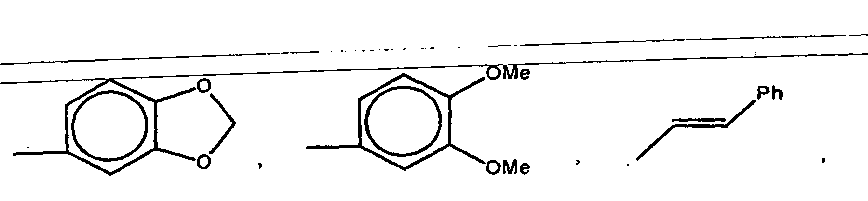 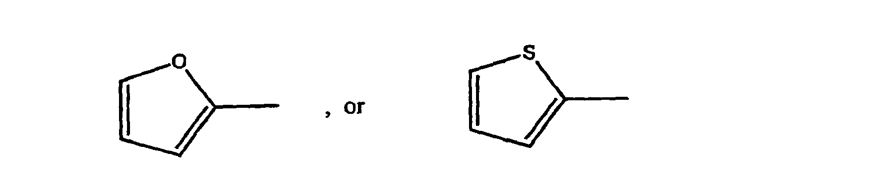 wherein X is as previously defined, Me is methyl and Ph is phenyl. Preferably, R₁ is selected from -OR₆, -SR₇ or -NR₈R₉ wherein R₆, R₇ and R₉, are hydroxy, sulfhydryl, and amine protecting groups, respectively, and R₈ is hydrogen, alkyl, alkenyl, alkynyl, aryl, or heteroaryl. Most preferably, R₁ is -OR₆ wherein R₆ is triethylsilyl ("TES"), 1-ethoxyethyl ("EE") or 2,2,2-trichloroethoxymethyl.

The β-lactam alkyl groups, either alone or with the various substituents defined hereinabove are preferably lower alkyl containing from one to six carbon atoms in the principal chain and up to 15 carbon atoms. They may be straight or branched chain and include methyl, ethyl, propyl, isopropyl, butyl, isobutyl, tert-butyl, amyl, hexyl, and the like.

The β-lactam alkenyl groups, either alone or with the various substituents defined hereinabove are preferably lower alkenyl containing from two to six carbon atoms in the principal chain and up to 15 carbon atoms. They may be straight or branched chain and include ethenyl, propenyl, isopropenyl, butenyl, isobutenyl, pentenyl, hexenyl, and the like.

The β-lactam alkynyl groups, either alone or with the various substituents defined hereinabove are preferably lower alkynyl containing from two to six carbon atoms in the principal chain and up to 15 carbon atoms. They may be straight or branched chain and include ethynyl, propynyl, butynyl, isobutynyl, pentynyl, hexynyl, and the like.

The β-lactam aryl moieties described, either alone or with various substituents, contain from 6 to 15 carbon atoms and include phenyl, α-naphthyl or β-naphthyl, etc. Substituents include alkanoxy, protected hydroxy, halogen, alkyl, aryl, alkenyl, acyl, acyloxy, nitro, amino, amido, etc. Phenyl is the more preferred aryl.

As noted above, R₁ of β-lactam (2) may be -OR₆ with R₆ being alkyl, acyl, ethoxyethyl ("EE"), triethylsilyl ("TES"), 2,2,2-trichloroethoxymethyl, or other hydroxyl protecting group such as acetals and ethers, i.e., methoxymethyl ("MOM"), benzyloxymethyl; esters, such as acetates; carbonates, such as methyl carbonates; and alkyl and aryl silyl such as triethylsilyl, trimethylsilyl, dimethyl-t-butylsilyl, dimethylarylsilyl, dimethylheteroarylsilyl, and triisopropylsilyl, and the like. A variety of protecting groups for the hydroxyl group and the synthesis thereof may be found in "Protective Groups in Organic Synthesis" by T. W. Greene, John Wiley and Sons, 1981. The hydroxyl protecting group selected should be easily removed under conditions that are sufficiently mild, e.g., in 48% HF, acetonitrile, pyridine, or 0.5% HCl/water/ ethanol, and/or zinc/acetic acid so as not to disturb the ester linkage or other substituents of the taxol intermediate. However, R₆ is preferably triethylsilyl, 1-ethoxyethyl or 2,2,2-trichloroethoxymethyl, and most preferably triethylsilyl.

Also as noted previously, R₇ may be a sulfhydryl protecting group and R₉ may be an amine protecting group. Sulfhydryl protecting groups include hemithioacetals such as 1-ethoxyethyl and methoxymethyl, or thiocarbonates. Amine protecting groups include carbamates, for example, 2,2,2-trichloroethylcarbamate or tertbutylcarbamate. A variety of sulfhydryl and amine protecting groups may be found in the above-identified text by T. W. Greene.

Since β-lactam (2) has several asymmetric carbons, it is known to those skilled in the art that the compounds of the present invention having asymmetric carbon atoms may exist in diastereomeric, racemic, or optically active forms. All of these forms are contemplated within the scope of this invention. More specifically, the present invention includes enantiomers, diastereomers, racemic mixtures, and other mixtures thereof.

β-lactams can be prepared from readily available materials, as is illustrated in schemes A and B below. Although the β-lactam prepared in the schemes is not in accordance with the present invention, the schemes provide useful background information. 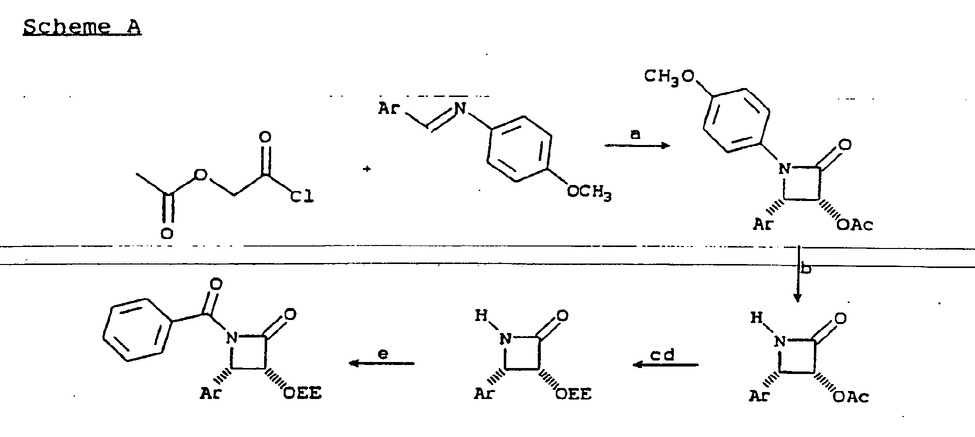 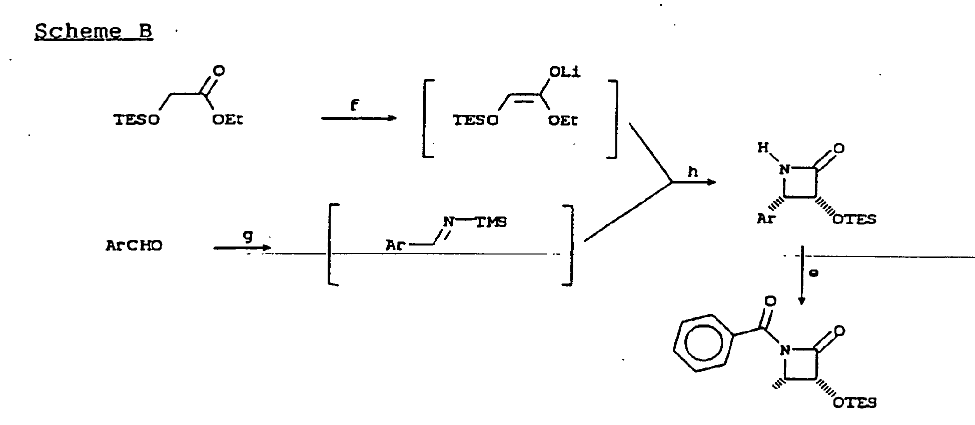 reagents: (a) triethylamine, CH₂Cl₂, 25°C, 18h; (b) 4 equiv ceric ammonium nitrate, CH₃CN, -10°C, 10 min; (c) KOH, THF, H₂O, O°C, 30 min; (d) ethyl vinyl ether, THF, toluene sulfonic acid (cat.), O°C, 1.5h; (e) n-butyllithium, ether, -78°C, 10 min; benzoyl chloride, -78°C, lh; (f) lithium diisopropyl amide, THF -78°C to -50°C; (g) lithium hexamethyldisilazide, THF -78°C to 0°C; (h) THF, -78°C to 25°C, 12h.

The starting materials are readily available. In scheme A, α-acetoxy acetyl chloride is prepared from glycolic acid, and, in the presence of a tertiary amine, it cyclocondenses with imines prepared from aldehydes and p-methoxyaniline to give 1-p-methoxyphenyl-3-acyloxy-4-arylazetidin-2-ones. The p-methoxyphenyl group can be readily removed through oxidation with ceric ammonium nitrate, and the acyloxy group can be hydrolyzed under standard conditions familiar to those experienced in the art to provide 3-hydroxy-4-arylazetidin-2-ones. The 3-hydroxyl group is protected with 1-ethoxyethyl, but may be protected with variety of standard protecting groups such as the triethylsilyl group or other trialkyl (or aryl) silyl groups. In Scheme B, ethyl-α-triethylsilyloxyacetate is readily prepared from glycolic acid.

The racemic β-lactams may be resolved into the pure enantiomers prior to protection by recrystallization of the corresponding 2-methoxy-2-(trifluoromethyl) phenylacetic esters. However, the reaction described hereinbelow in which the β-amido ester side chain is attached has the advantage of being highly diastereoselective, thus permitting the use of a racemic mixture of side chain precursor.

The 3-(1-ethoxyethoxy)-4-phenylazetidin-2-one of scheme A and the 3-(i-triethylsilyloxy)-4-phenylazetidin-2-one of scheme B can be converted to β-lactam (2), by treatment with a base, preferably n-butyllithium, and an acyl chloride, alkylchloroformate, sulfonyl chloride, phosphinyl chloride or phosphoryl chloride at -78 °C or below.

The process of the present invention is particularly useful for the esterification of mono- or polycyclic metal alkoxides which are represented by the formula 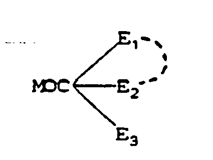 in which E₁, E₂ and the carbon to which they are attached define a carbocyclic and/or heterocyclic skeleton which may be mono- or polycyclic and E₃ is hydrogen or hydrocarbon, preferably lower alkyl. Most preferably, the carbocyclic and/or heterocyclic skeleton comprises about 6 to 20 atoms and the hetero atoms are oxygen. The cyclic skeleton may be hydrocarbon and/or heterosubstituted with heterosubstituents including, for example, esters, ethers, amines, alcohols, protected alcohols, carbonyl groups, halogens, oxygen, substituted oxygen or substituted nitrogen.

When the metal alkoxides have the bi-, tri- or tetracyclic taxane nucleus, the process of the present invention may advantageously be used to prepare taxane derivatives, many of which have been found to have significant biological activity. As used herein, a metal alkoxide having the bicyclic taxane nucleus has the carbocyclic skeleton corresponding to rings A and B of metal alkoxide (3): 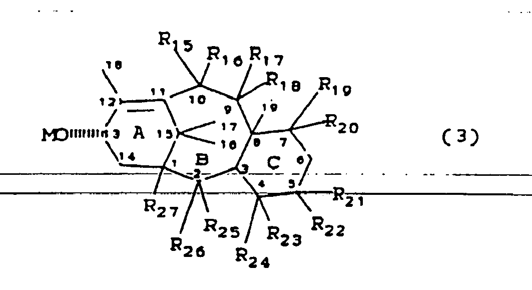 M and R₁₅-R₂₇ are as previously defined. A metal alkoxide having the tricyclic taxane nucleus has the carbocyclic skeleton corresponding to rings A, B and C of metal alkoxide (3). A metal alkoxide having the tetracyclic taxane nucleus has carbocyclic rings A, B and C of metal alkoxide (3) and the oxetane ring defined by R₂₂, R₂₃, and the carbons to which they are attached.

Preferably, the metal alkoxide used in the process of the present invention is metal alkoxide (3). Most preferably, R₁₅ is -OT₂ or -OCOCH₃; R₁₆ is hydrogen; R₁₇ and R₁₈ together form an oxo; R₁₉ is -OT₁; R₂₀ and R₂₁ are hydrogen; R₂₂ and R₂₃ together with the carbons to which they are attached form an oxetane ring; R₂₄ is CH₃COO-; R₂₅ is PhCOO-; R₂₆ is hydrogen; R₂₇ is hydroxy; and T₁ and T₂ are independently hydrogen or hydroxy protecting group.

Metal substituent, M, of metal alkoxide (3) is a Group IA, IIA, IIIA, lanthanide or actinide element or a transition, Group IIIA, IVA, VA or VIA metal. Preferably, it is a Group IA, IIA or transition metal, and most preferably, it is lithium, magnesium, sodium, potassium or titanium.

The metal alkoxide alkyl groups, either alone or with the various substituents defined hereinabove are preferably lower alkyl containing from one to six carbon atoms in the principal chain and up to 10 carbon atoms. They may be straight or branched chain and include methyl, ethyl, propyl, isopropyl, butyl, isobutyl, tert-butyl, amyl, hexyl, and the like.

The metal alkoxide alkenyl groups, either alone or with the various substituents defined hereinabove are preferably lower alkenyl containing from two to six carbon atoms in the principal chain and up to 10 carbon atoms. They may be straight or branched chain and include ethenyl, propenyl, isopropenyl, butenyl, isobutenyl, pentenyl, hexenyl, and the like.

The metal alkoxide alkynyl groups, either alone or with the various substituents defined hereinabove are preferably lower alkynyl containing from two to six carbon atoms in the principal chain and up to 10 carbon atoms. They may be straight or branched chain and include ethynyl, propynyl, butynyl, isobutynyl, pentynyl, hexynyl, and the like.

Exemplary alkanoyloxy include acetate, propionate, butyrate, valerate, isobutyrate and the like. The more preferred alkanoyloxy is acetate.

The metal alkoxide aryl moieties, either alone or with various substituents contain from 6 to 10 carbon atoms and include phenyl, α-naphthyl or β-naphthyl, etc. Substituents include alkanoxy, hydroxy, halogen, alkyl, aryl, alkenyl, acyl, acyloxy, nitro, amino, amido, etc. Phenyl is the more preferred aryl.

Metal alkoxides (3) are prepared by reacting an alcohol having two to four rings of the taxane nucleus and a C-13 hydroxyl group with an organometallic compound in a suitable solvent. Preferably, the alcohol is a derivative of baccatin III or 10-deacetyl baccatin III having the structure 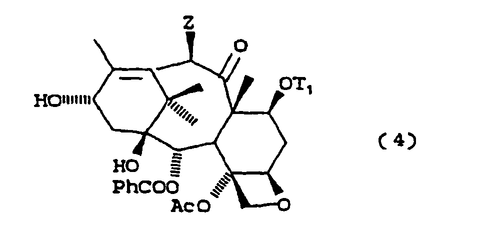 wherein T₁ is a hydroxy protecting group, and Z is -OT₂ wherein T₂ is acyl, preferably acetyl, or other hydroxy protecting group. Most preferably, the alcohol is a protected baccatin III, in particular, 7-O-triethylsilyl baccatin III (which can be obtained as described by Greene, et al. in JACS 110, 5917 (1988) or by other routes) or 7,10-bis-O-triethylsilyl baccatin III.

As reported in Greene et al., 10-deacetyl baccatin III is converted to 7-O-triethylsilyl-10-deacetyl baccatin III according to the following reaction scheme: 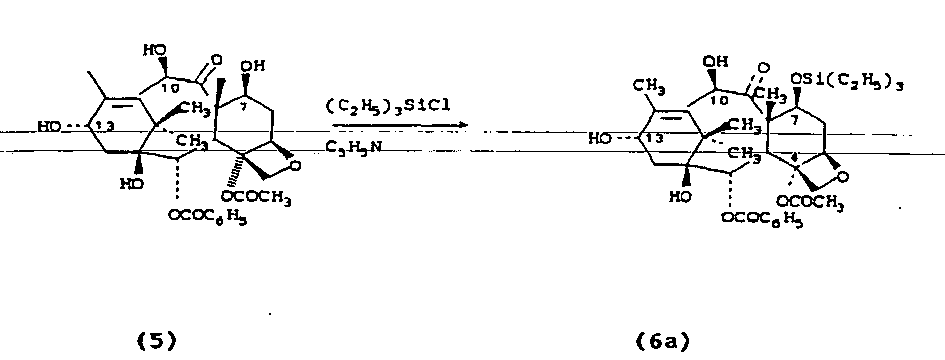 Under what is reported to be carefully optimized conditions, 10-deacetyl baccatin III is reacted with 20 equivalents of (C₂H₅)₃SiCl at 23°C under an argon atmosphere for 20 hours in the presence of 50 ml of pyridine/mmol of 10-deacetyl baccatin III to provide 7-triethylsilyl-10-deacetyl baccatin III **(6a)** as a reaction product in 84-86% yield after purification. The reaction product is then acetylated with 5 equivalents of CH₃COCl and 25 mL of pyridine/mmol of **(6a)** at 0 °C under an argon atmosphere for 48 hours to provide 86% yield of 7-O-triethylsilyl baccatin III **(6b).** Greene, et al. in JACS 110, 5917 at 5918 (1988).

Alternatively, 7-triethylsilyl-10-deacetyl baccatin III **(6a)** can be protected at C-10 oxygen with an acid labile hydroxyl protecting group. For example, treatment of **(6a)** with n-butyllithium in THF followed by triethylsilyl chloride (1.1 mol equiv.) at 0°C gives 7,10-bis-O-triethylsilyl baccatin III **(6c)** in 95% yield. Also, **(6a)** can be converted to 7-O-triethylsilyl-10-(1-ethoxyethyl) baccatin III **(6d)** in 90% yield by treatment with excess ethyl vinyl ether and a catalytic amount of methane sulfonic acid. These preparations are illustrated in the reaction scheme below.

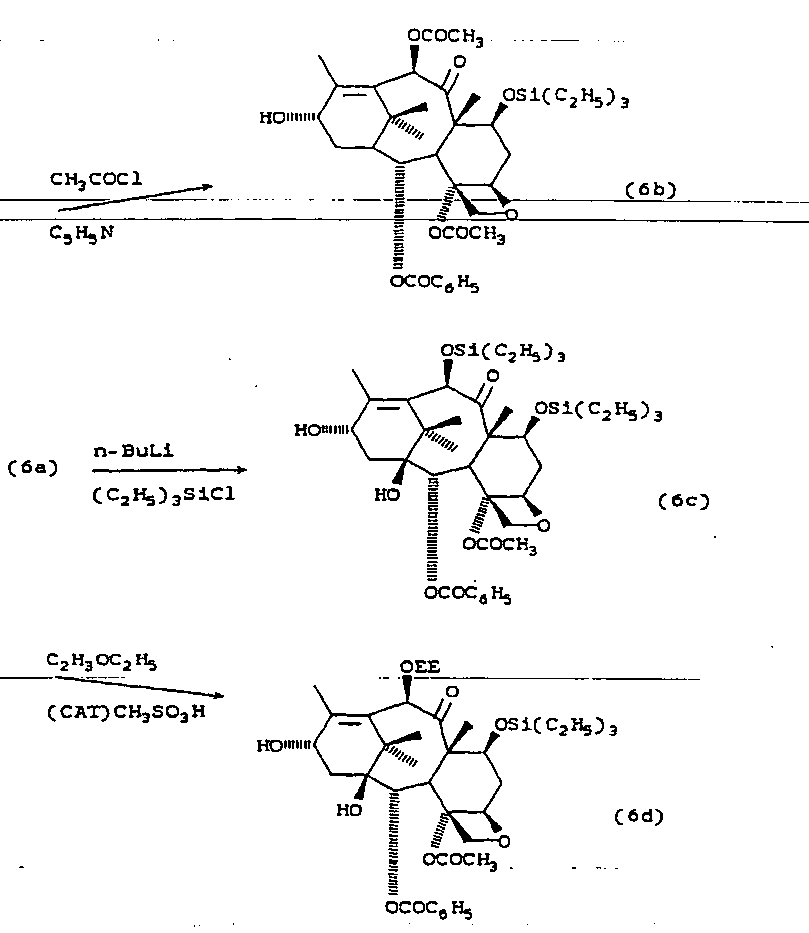

7-O-triethylsilyl baccatin III **(6b)**, 7,10-bis-0-triethylsilyl baccatin III **(6c),** or 7-O-triethylsilyl-10-(1-ethoxyethyl) baccatin III **(6d)** is reacted with an organometallic compound such as n-butyllithium in a solvent such as tetrahydrofuran (THF), to form the metal alkoxide 13-O-lithium-7-O-triethylsilyl baccatin III **(7b)** 13-O-lithium-7,10-bis-O-triethylsilyl baccatin III **(7c),** or 13-O-lithium-7-O-triethylsilyl-10-(1-ethoxyethyl) baccatin III **(7d)** as shown in the following reaction scheme:

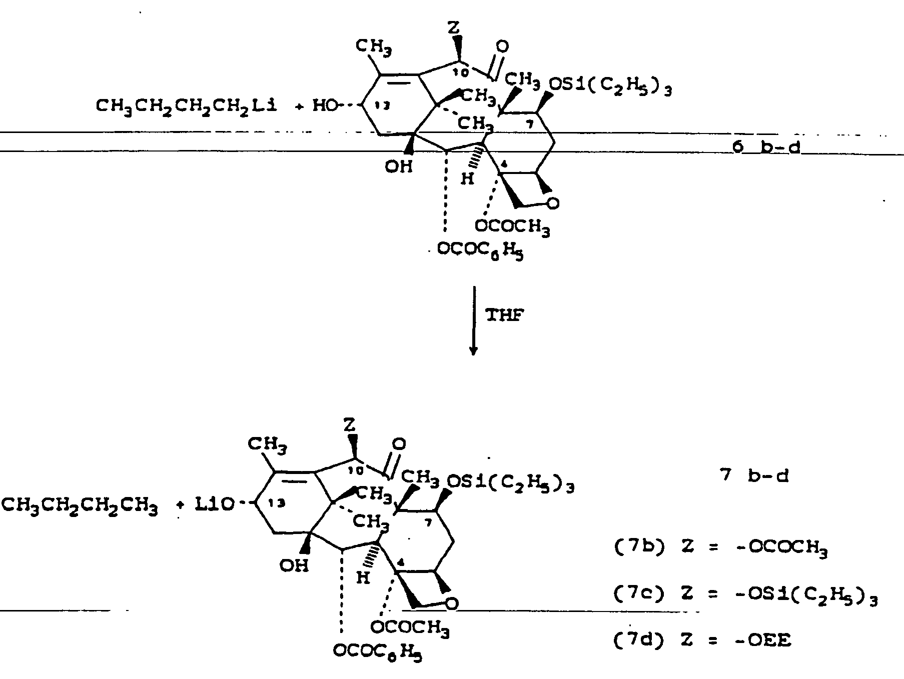

As illustrated in the following reaction scheme, a suitable metal alkoxide of the present invention such as 13-O-lithium-7-O-triethylsilyl baccatin III derivative **(7b, 7c,** or **7d)** reacts with a β-lactam of the present invention to provide an intermediate **(8b, 8c,** or **8d)** in which the C-7 hydroxyl group is protected with a triethylsilyl or 1-ethoxyethyl group.

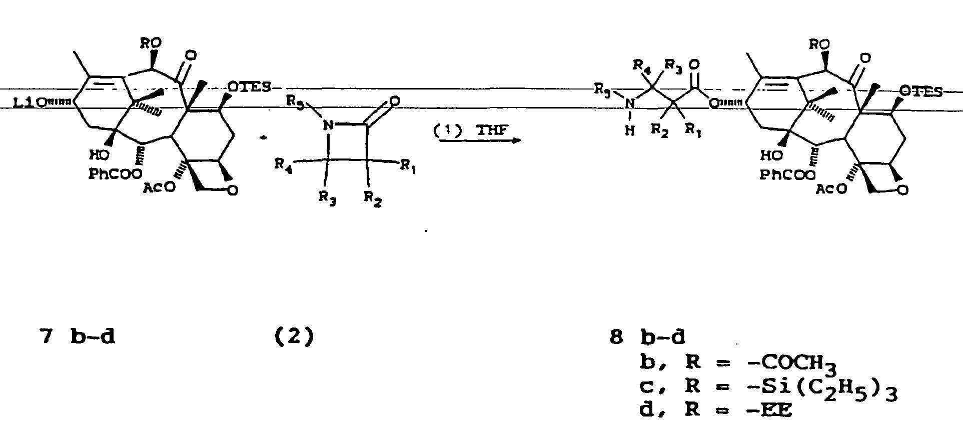

Intermediate compound **(8b)** readily converts to taxol when R₁ is -OR₆, R₂ and R₃ are hydrogen, R₄ is phenyl, R₅ is benzoyl and R₆ is a hydroxy protecting group such as triethylsilyl. Intermediate compound (8c) readily converts to taxotere when R₁ is -OR₆, R₂ and R₃ are hydrogen, R₄ is phenyl, R₅ is tertbutoxycarbonyl and R₆ is a hydroxy protecting group such as triethylsilyl. Intermediate compound **(8d)** readily converts to 10-deacetyl taxol when R₁ is -OR₆, R₂ and R₃ are hydrogen, R₄ is phenyl, R₅ is benzoyl, and R₆ is a hydroxy protecting group such as triethylsilyl. Intermediate compounds **(8b, 8c** and **8d)** may be converted to the indicated compounds by hydrolyzing the triethylsilyl and 1-ethoxyethyl groups under mild conditions so as not to disturb the ester linkage or the taxane derivative substituents.

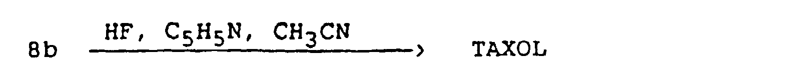

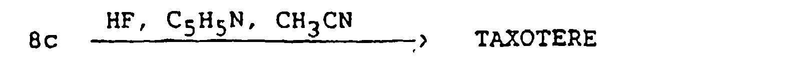

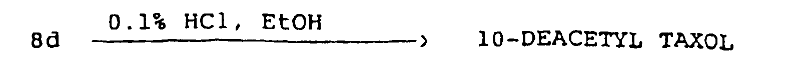

Other taxane derivatives may readily be prepared by selection of the proper substituents R₁-R₅ of β-lactam (2) or R₁₅ - R₂₇ of metal alkoxide (3). The preparation of such other compounds is illustrated in the examples which follow.

Both the conversion of the alcohol to the metal alkoxide and the ultimate synthesis of the taxol can take place in the same reaction vessel. Preferably, the β-lactam is added to the reaction vessel after formation therein of the metal alkoxide.

The organometallic compound n-butyllithium is preferably used to convert the alcohol to the corresponding metal alkoxide, but other sources of metallic substituent such as lithium diisopropyl amide, other lithium or magnesium amides, ethylmagnesium bromide, methylmagnesium bromide, other organolithium compounds, other organomagnesium compounds, organosodium, organotitanium, organozirconium, organozinc, organocadmium or organopotassium or the corresponding amides may also be used. Organometallic compounds are readily available, or may be prepared by available methods including reduction of organic halides with metal. Lower alkyl halides are preferred. For example, butyl bromide can be reacted with lithium metal in diethyl ether to give a solution of n-butyllithium in the following manner:

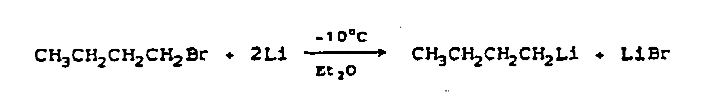

Alternatively, the lithium alkoxide may be induced to undergo exchange with metal halides to form alkoxides of aluminum, boron, cerium, calcium, zirconium or zinc.

Although THF is the preferred solvent for the reaction mixture, other ethereal solvents, such as dimethoxyethane, or aromatic solvents may also be suitable. Certain solvents, including some halogenated solvents and some straight-chain hydrocarbons in which the reactants are too poorly soluble, are not suitable. Other solvents are not appropriate for other reasons. For example, esters are not appropriate for use with certain organometallic compounds such as n-butyllithium due to incompatibility therewith.

Although the reaction scheme disclosed herein is directed to the synthesis of certain taxol derivatives, it can be used with modifications in either the β-lactam or the tetracyclic metal alkoxide. Therefore metal alkoxides other than 13-O-lithium-7-O-triethylsilyl baccatin III may be used to form a taxol intermediate according to the method of this invention. The β-lactam and the tetracyclic metal alkoxide can be derived from natural or unnatural sources, to prepare other synthetic taxols, taxol derivatives, 10-deacetyltaxols, and the enantiomers and diastereomers thereof contemplated within the present invention.

The process of the invention also has the important advantage of being highly diastereoselective. Therefore racemic mixtures of the side chain precursors may be used. Substantial cost savings may be realized because there is no need to resolve racemic β-lactams into their pure enantiomers. Additional cost savings may be realized because less side chain precursor, e.g., 60-70% less, is required relative to prior processes.

The water solubility of compounds of formula (1) may be improved if R₁ is -OR₆ and R₁₉ is -OT₁, and R₆ and/or T₁ are a functional group which increases solubility, such as -COGCOR¹ wherein:
- G: is ethylene, propylene, CHCH, 1,2-cyclohexylene, or 1,2-phenylene;
- R₁ =: OH base, NR²R³, OR³, SR³, OCH₂CONR⁴R⁵, or OH;
- R₂ =: hydrogen or methyl;
- R₃ =: (CH₂)ₙNR⁶R⁷; or (CH₂)ₙN^{⊕}R⁶R⁷R⁸X₁^{⊖}
- n =: 1 to 3;
- R⁴ =: hydrogen or lower alkyl containing 1 to 4 carbons;
- R⁵ =: hydrogen, lower alkyl containing 1 to 4 carbons, benzyl, hydroxyethyl, CH₂CO₂H, or dimethylaminoethyl;
- R⁶ and R⁷ =: lower alkyl containing 1 or 2 carbons, or benzyl; or
- R⁶ and R⁷: together with the nitrogen atom of NR⁶R⁷ forms one of the following rings 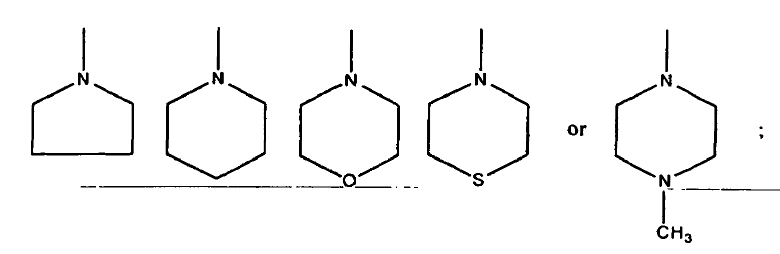
- R⁸ =: lower alkyl containing 1 or 2 carbons or benzyl;
- x₁^{⊖} =: halide; and
- base =: NH₃, (HOC₂H₄)₃N, N(CH₃)₃, CH₃N(C₂H₄OH)₂, NH₂(CH₂)₆NH₂, N-methylglucamine, NaOH or KOH.
The preparation of compounds in which R₆ or T₁ is -COGCOR¹ is set forth in Haugwitz U.S. Patent 4,942,184 which is incorporated herein by reference.

The following examples illustrate the invention.

### EXAMPLE 1

### Preparation of taxotere.

To a solution of 7,10-bis-triethylsilyl baccatin III (200 mg, 0.248 mmol)) in 2 mL of THF at -45 "C was added dropwise 0.174 mL of a 1..63M solution of nBuLi in liexane. After 0.5 h at -45 OC, a solution of cis-1-(tert butoxycarbonyl) -3-triethylsi lyloxy-4-phenylazetidin-2-one (467 mg, 1.24 mmol) in 2 mL of THF was added dropwise to the mixture. The solution was warmed to 0 OC and kept at that temperature for 1 h before 1 mL of a 10% solution of AcOH in THF- was added. The mixture was partitioned between saturated aqueous NaHCO3 and 60/40 ethyl acetate/hexane.

Evaporation of the organic layer gave a residue which was purified by filtration through silica gel to give 280 mg of crude 2' ,7,10-tris-triethylsilyl taxotere.

To a solution of 280 mg of the crude product obtained from the previous reaction in 12 mL of acetonitrile and 0.6 mL of pyridine at 0 "C was added 1.8 mL of 48% aqueous HF. The mixture was stirred at 0 "C for 3 h, then at 25 OC for 13 h, and partitioned between saturated aqueous sodium bicarbonate and ethyl acetate.

Evaporation of the ethyl acetate solution gave 215 mg of material which was purified by flash chromatography to give 190 mg (95%) of taxotere, which was recrystallized from methanol/water. All analytical and spectral data were identical with that reported for taxotere in U.S. Patent 4,814,470.

### EXAMPLE 2

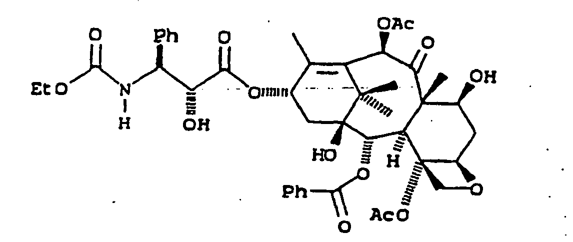

Preparation of N-debenzoyl-N-ethoxycarbonyl taxol.

To a solution of 7-triethylsilyl baccatin III (155 mg, 0.221 mmol) in 2 mL of THF at -45 OC was added dropwise 0.136 mL of a 1.63M solution of nBuLi in hexane.

After 0.5 h at -45 OC, a solution of cis-1-ethoxycarbonyl- 3-triethylsilyloxy-4-phenylazetidin-2-one (386 mg, 1.11 mmol) in 2 mL of THF was added dropwise to the mixture.

The solution was warmed to 0 OC and kept at that temperature for 1 h before 1 mL of a 10% solution of AcOH in THF was added. The mixture was partitioned between saturated aqueous NaHC03 and 60/40 ethyl acetate/hexane.

Evaporation of the organic layer gave a residue which was purified by filtration through silica gel to give 252 mg of a mixture containing (2'R,3'S)-2' ,7-(bis)triethylsilyl-N-debenzoyl-N-ethoxycarbonyl taxol and a small amount of the (2'S,3'R) isomer.

To a solution of 252 mg (0.112 mmol) of the mixture obtained from the previous reaction in 12 mL of acetonitrile and 0.6 mL of pyridine at'0 "C was added 1.8 mL of 48% aqueous HF. The mixture was stirred at 0 "C for 3 h, then at 25 OC for 13 h, and partitioned between saturated aqueous sodium bicarbonate and ethyl acetate.

Evaporation of the ethyl acetate solution gave 216 mg of material which was purified by flash chromatography to give 155 mg (85%) of N-debenzoyl-N-ethoxycarbonyl taxol, which was recrystallized from methanol/water.

m.p. 161.5-162.5 °C; [α]²⁵_{Na}-62.2° (c 0.51, CHCl₃).

¹H NMR (CDCl₃, 300 MHz) δ 8.12 (d, J = 7.7 Hz, 2H, benzoate ortho), 7.65-7.3 (m, 8H, aromatic), 6.28 (m, 1H, H10) 6.27(m, 1H, H13), 5.67 (d, J = 7.1 Hz, 1H, H2β) 5.53 (d, J = 9.3 Hz, 1H, H3'), 5.29 (d, J = 9.3 Hz, 1H, NH), 4.94 (dd, J = 9.3, 2.2 Hz, 1H, H5), 4.64 (dd, J = 5.0, 2.8 Hz, 1H, H2'), 4.41 (m, 1H, H7), 4.29 (d, J = 8.5 Hz, 1H, H20α), 4.17 (d, J = 8.5 Hz, 1H, H20β, 4.01 (q, J = 7.1 Hz, 2H, COOCH₂CH₃), 3.79 (d, J = 7.1 Hz, 1H, H3), 3.45 (d, J = 5 Hz, 1H, 2'OH), 2.54 (m, 1H, H6α), 2.47 (d, J = 3.9 Hz 1H, 70H), 2.36 (s, 3H, 4Ac),2.24 (s, 3H, 10Ac), 2.22 (m, 2H, H14α, H14β), 1.87 (m, 1H, H6α), 1.83 (br s, 3H, Me18), 1.77 (s, 1H, 10H), 1.68 (s, 3H, Me19), 1.27 (s, 3H, Me17), 1.15 (s, 3H, Me16), 1.14 (t, J = 7.1 Hz, 2H, COOCH₂CH₃).

In view of the above, it will be seen that several objects of the invention are achieved.

As various changes could be made in the above compositions and processes without departing from the scope of the invention, it is intended that all matter contained in the above description be interpreted as illustrative and not in a limiting sense.

## Claims

1. A β-lactam having the formula: 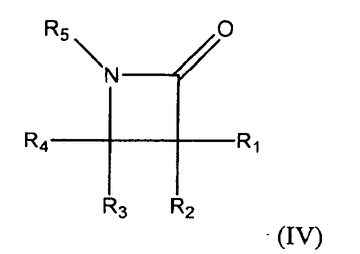 wherein
R₁ is -OR₆, -SR₇, or -NR₈R₉;
R₂ is hydrogen, alkyl, alkenyl, alkynyl, aryl, or heteroaryl;
R₃ and R₄ are independently hydrogen, alkyl, alkenyl, alkynyl, aryl, heteroaryl, or acyl, provided, however, that R₃ and R₄ are not both acyl;
R₅ is -COOR₁₀, -CONR₈R₁₀, or -S0₂₁₁;
R6 is hydrogen, alkyl, alkenyl, alkynyl, aryl, heteroaryl, hydroxy protecting group, or a functional group which increases the water solubility of a taxane derivative; R₇ is alkyl, alkenyl, alkynyl, aryl, heteroaryl, or sulfhydryl protecting group;
R₈ is hydrogen, alkyl, alkenyl, alkynyl, aryl, or heteroaryl;
R₉ is an amino protecting group;
R₁₀ is alkyl;
R₁₁ is alkyl, alkenyl, alkynyl, aryl, heteroaryl, -OR₁₀, or -NR₈R₁₄; and
R₁₄ is hydrogen, alkyl, alkenyl, alkynyl, aryl, or heteroaryl.

2. A β-lactam according to Claim 1 wherein R₂ and R₄ are hydrogen or lower alkyl.

3. A β-lactam according to Claim 1 wherein R₃ is aryl.

4. A β-lactam according to Claim 3 wherein the aryl is naphtyl, phenyl, 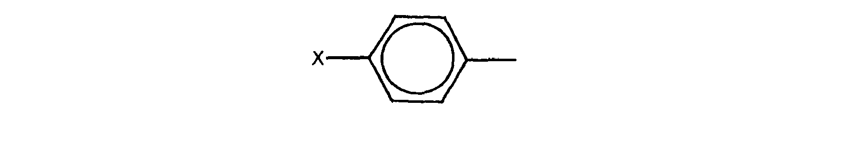 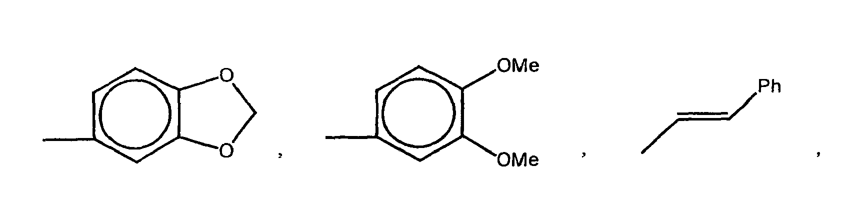 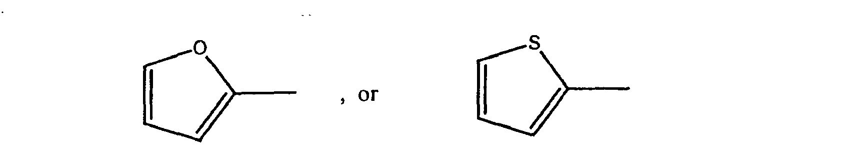 wherein X is Cl, Br, F, Br, F, CH₃O- or NO₂-, Me is methyl and Ph is phenyl.

5. A β-lactam according to Claim 1 wherein R₆, R₇ and R₉ are hydroxy, sulfhydryl and amine groups respectively.

6. A β-lactam according to Claim 1 wherein R₆ is triethylsilyl, 1-ethoxyethyl or 2,2,2-trichloroethoxymethyl.

7. A β-lactam according to Claim 1 wherein R₉ is 2,2,2-trichloroethylcarbamate or tertbutylcarbamate.

## Patentansprüche

1. β-Lactam mit der folgenden Formel: 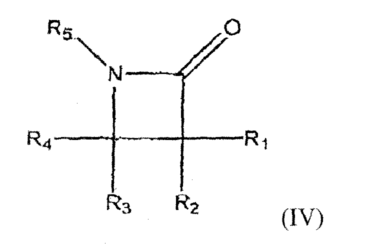 wobei
R₁ für -OR₆, -SR₇ oder NR₈R₉ steht;
R₂ für Wasserstoff, Alkyl, Alkenyl, Alkinyl, Aryl oder Heteroaryl steht;
R₃ und R₄ unabhängig Wasserstoff, Alkyl, Alkenyl, Alkinyl, Aryl, Heteroaryl oder Acyl sind, mit der Maßgabe jedoch, dass R₃ und R₄ nicht beide Acyl sind;
R₅ für -COOR₁₀, -CONR₈R₁₀ oder -SO₂R₁₁ steht;
R₆ für Wasserstoff, Alkyl, Alkenyl, Alkinyl, Aryl, Heteroaryl, Hydroxyschutzgruppe oder eine funktionelle Gruppe, welche die Wasserlöslichkeit eines Taxanderviats erhöht, steht;
R₇ für Alkyl, Alkenyl, Alkinly, Aryl, Heteroaryl oder Sulfhydrylschutzgruppe steht;
R₈ für Wasserstoff, Alkyl, Alkenyl, Alkinyl, Aryl oder Heteroaryl steht;
R₉ eine Aminoschutzgruppe ist;
R₁₀ für Alkyl steht;
R₁₁ für Alkyl, Alkenyl, Alkinyl, Aryl, Heteroaryl, -OR₁₀ oder -NR₈R₁₄ steht; und
R₁₄ für Wasserstoff, Alkyl, Alkenyl, Alkinyl, Aryl oder Heteroaryl steht.

2. β-Lactam gemäß Anspruch 1, wobei R₂ und R₄ für Wasserstoff oder Niederalkyl stehen.

3. β-Lactam gemäß Anspruch 1, wobei R₃ für Aryl steht.

4. β-Lactam gemäß Anspruch 3, wobei das Aryl für Naphthyl, Phenyl, 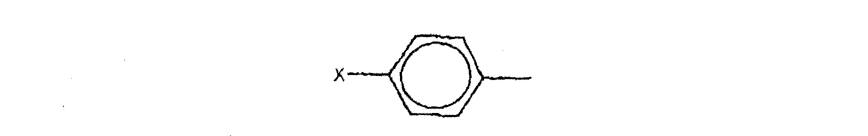 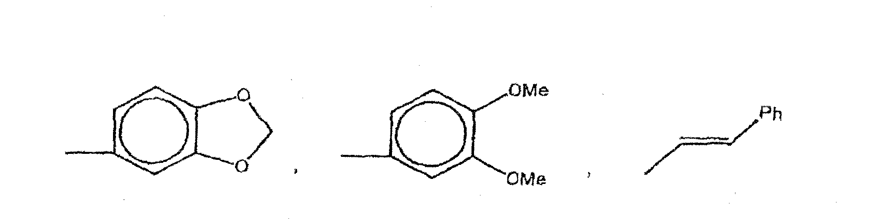 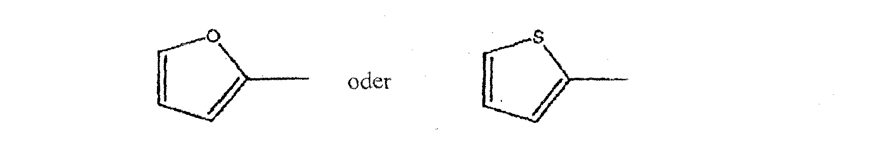 steht, wobei X für Cl, Br, F, Br, F, CH₃O- oder NO₂- steht, Me für Methyl steht und Ph für Phenyl steht.

5. β-Lactam gemäß Anspruch 1, wobei R₆, R₇ und R₉ für Hydroxy-, Sulfhydrylbeziehungsweise Amingruppen stehen.

6. β-Lactam gemäß Anspruch 1, wobei R₆ für Triethylsilyl, 1-Ethoxyethyl oder 2,2,2-Trichlorethoxymethyl steht.

7. β-Lactam gemäß Anspruch 1, wobei R₉ für 2,2,2-Trichlorethylcarbamat oder tert-Butylcarbamat steht.

## Revendications

1. β-lactame ayant la formule : 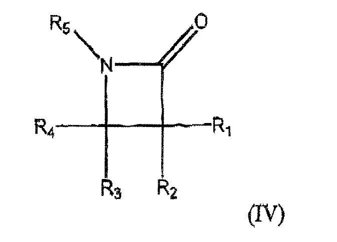 dans laquelle
R₁ est -OR₆, -SR₇ ou -NR₈R₉ ;
R₂ est un atome d'hydrogène, un groupe alkyle, alcényle, alcynyle, aryle ou hétéroaryle ;
R₃ et R₄ sont indépendamment un atome d'hydrogène, un groupe alkyle, alcényle, alcynyle, aryle, hétéroaryle ou acyle, à condition, toutefois, que R₃ et R₄ ne sont pas tous les deux un groupe acyle ;
R₅ est -COCR₁₀, -CONR₈R₁₀ ou -SO₂R₁₁ ;
R₆ est un atome d'hydrogène, un groupe alkyle, alcényle, alcynyle, aryle, hétéroaryle, un groupe hydroxy-protecteur ou un groupe fonctionnel qui augmente l'hydrosolubilité d'un dérivé de taxane ;
R₇ est un groupe alkyle, alcényle, alcynyle, aryle, hétéroaryle ou sulfhydryl-protecteur ;
R₈ est un atome d'hydrogène, un groupe alkyle, alcényle, alcynyle, aryle ou hétéroaryle ;
R₉ est un groupe amino-protecteur;
R₁₀ est un groupe alkyle ;
R₁₁ est un groupe alkyle, alcényle, alcynyle, aryle, hétéroaryle, -OR₁₀ ou -NR₈R₁₄ ; et
R₁₄ est atome d'hydrogène, un groupe alkyle, alcényle, alcynyle, aryle ou hétéroaryle.

2. β-lactame selon la revendication 1 dans lequel R₂ et R₄ sont un atome d'hydrogène ou un groupe alkyle inférieur.

3. β-lactame selon la revendication 1 dans lequel R₃ est un groupe aryle.

4. β-lactame selon la revendication 3 dans lequel le groupe aryle est un radical naphtyle, phényle, 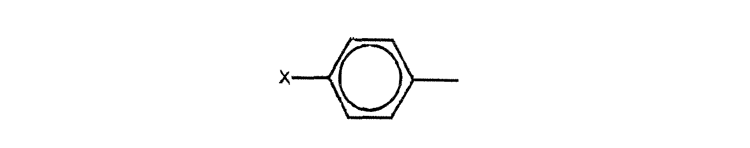 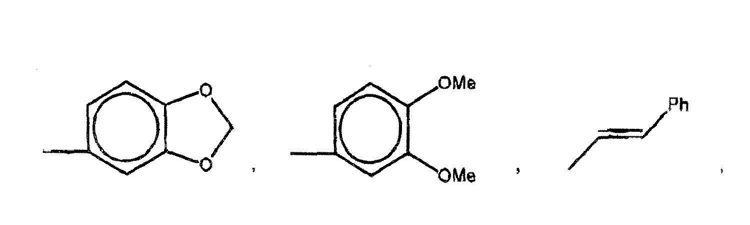 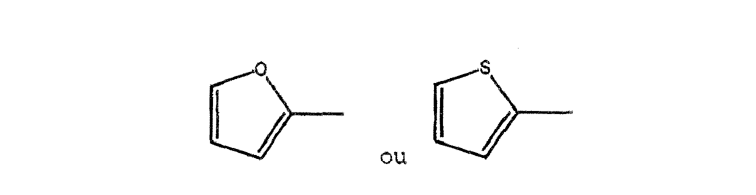 où X est Cl, Br, F, Br, F, CH₃O- ou NO₂-, Me est un radical méthyle et Ph est un radical phényle.

5. β-lactame selon la revendication 1 dans lequel R₆, R₇ et R₉ sont des groupes hydroxy, sulfhydryle et amine, respectivement.

6. β-lactame selon la revendication 1 dans lequel R₆ est le triéthylsilyle, 1-éthoxyéthyle ou 2,2,2-trichloroéthoxyméthyle.

7. β-lactame selon la revendication 1 dans lequel R₉ est le 2,2,2-trichloroéthylcarbamate ou tertbutylcarbamate.
